# EUROPEAN PATENT SPECIFICATION

(11) **EP 3 768 381 B1**
(45) Date of publication and mention of the grant of the patent: **08.03.2023**
(21) Application number: 19715303.4
(22) Date of filing: 19.03.2019
(51) Int. Cl.: A61N 5/06

(54) **AN OPTICAL STIMULATION SYSTEM WITH AUTOMATED MONITORING**
OPTISCHES STIMULATIONSSYSTEM MIT AUTOMATISCHER ÜBERWACHUNG
SYSTÈME DE STIMULATION OPTIQUE DOTÉ D'UNE SURVEILLANCE AUTOMATISÉE

(30) Priority: 23.03.2018 US 201862647538 P
(43) Date of publication of application: 27.01.2021
(73) Proprietor: Boston Scientific Neuromodulation Corporation, Valencia, CA 91355 (US); Commissariat à l'Energie Atomique et aux Energies Alternatives (CEA), 75015 Paris (FR)
(72) Inventor: FEATHERSTONE, Adam, Thomas, Meridian, ID 83646 (US); FELDMAN, Emanuel, Simi Valley, CA 93063 (US); RIVERA, John, Oxnard, CA 93036 (US); CHABROL, Claude, 38320 Poisat (FR); VANSICKLE, Dennis, Allen, Lancaster, CA 93536 (US); MOFFITT, Michael, A., Solon, OH 44139 (US); RENAULT, Sarah, 38700 Corenc (FR); POIZAT, Adrien, 38500 Voiron (FR)
(74) Representative: Vossius & Partner Patentanwälte Rechtsanwälte mbB
(86) International application number: PCT/US2019/022945
(87) International publication number: WO 2019/183075

(56) References cited:
- WO-A1-00/15101
- WO-A1-2011/112931
- US-A1- 2009 054 955
- US-A1- 2011 029 041
- US-A1- 2013 102 861
- US-A1- 2017 281 966
- US-A1- 2017 361 122

## Description

### FIELD

The present disclosure is directed to the area of implantable optical stimulation systems.

### BACKGROUND

Implantable optical stimulation systems can provide therapeutic benefits in a variety of diseases and disorders. For example, optical stimulation can be applied to the brain either externally or using an implanted stimulation lead to provide, for example, deep brain stimulation, to treat a variety of diseases or disorders. Optical stimulation may also be combined with electrical stimulation.

Stimulators have been developed to provide therapy for a variety of treatments. A stimulator can include a control module (for generating light or electrical signals sent to light sources in a lead), one or more leads, and one or more light sources coupled to, or disposed within, each lead. The lead is positioned near the nerves, muscles, brain tissue, or other tissue to be stimulated. For example, US 2013/0102861 discloses a medical apparatus that includes a light source unit configured to be capable of emitting light including at least natural emission light, the light being guided into an optical probe; a first detecting unit configured to detect an intensity of light exiting from the light source unit; and a calculation unit configured to calculate to approximate to a non-linear function based on an intensity of light detected by the first detecting unit for calibration of the optical probe.

US 2017/0281966 discloses a device to kill microorganisms inside the patient's respiratory tract using UV light, specifically UV-C. The device consists of a triple-lumen catheter and UV control unit. The distal end of one of the catheter's lumen has a curved UV chamber while the proximal end has a cable with a socket to be connected to the UV control unit. The second lumen of the catheter is used for oxygen supply, and the third lumen of the catheter is used for suction.

Document US 2017/361122 discloses an implantable optical stimulation device wherein the light source is connected to optical fibers. Further, there is a monitoring probe and a light detector, for measuring light injected into the tissue. It is then possible to ensure that the device is properly working and to check the light dose applied to the individual.

Document WO 00/15101 discusses a light-emitting catheter, wherein the same optical lead is connected to the light source, to guide the emitted light into the tissue and to the light monitor.

US 2011/029041 discloses a hearing aid implant comprising an optical microphone, comprising a light source, a light monitor and an optical lead coupling the source and the monitor. The monitor transforms the optical signal into an electrical signal.

### BRIEF SUMMARY

The optical stimulation system includes a light source configured to produce light for optical stimulation; a light monitor; an optical lead coupled, or coupleable, to the light source and the light monitor; and a control module coupled, or coupleable, to the light source and the light monitor. The light source, optical lead, control module and the light monitor are implantable. The control module includes a memory, and a processor coupled to the memory and configured for automatically initiating a verification or measurement of a light output value; in response to the initiation, receiving, from the light monitor, a measurement of light generated by the light source; and, when the measurement deviates from an expected light output value by more than a threshold amount, performing at least one of the following: sending a warning; or taking a corrective action.

In at least some aspects, the processor is further configured for directing the light monitor to make the measurement. In at least some aspects, the processor is further configured for directing the light source to generate light that is expected to be at the expected light output level at a site where light is collected for measurement by the light monitor. In at least some aspects, the light monitor is configured to measure a light output level directly from the light source. In at least some aspects, the optical lead further includes a first optical waveguide configured to receive light generated by the light source and emit the light from a distal portion of the optical lead for the optical stimulation and a second optical waveguide configured to receive a portion of the light emitted from the distal portion of the optical lead and direct the received portion of the light to the light monitor, wherein the light monitor is configured to measure a light output level from the light emitted from the distal portion of the optical lead.

In at least some aspects, the optical stimulation system further includes an external device configured for communication with the control module, wherein the processor is configured to send the warning to the external device and the external device is configured for, in response to receiving the warning, providing a visual, vibratory, or audible message to a user. In at least some aspects, the external device is a programming unit, a clinician programmer, or a patient remote control. In at least some aspects, the corrective action includes prompting or directing a user, through the external device, to adjust the optical stimulation if the measurement deviates by more than a threshold amount from an expected light output level.

In at least some aspects, sending a warning includes causing the control module to emit a vibratory or auditory warning. In at least some aspects, the corrective action includes prompting or directing a user to adjust the optical stimulation. In at least some aspects, the corrective action further includes, after the user adjusts the optical stimulation, receiving, from the light monitor, a second measurement of light generated by the light source; and, when the second measurement deviates from an expected light output value by more than a threshold amount, prompting or directing the user to adjust the optical stimulation.

In at least some aspects, the corrective action includes automatically adjusting the optical stimulation. In at least some aspects, the corrective action further includes, after automatically adjusting the optical stimulation, receiving, from the light monitor, a second measurement of light generated by the light source; and, when the second measurement deviates from an expected light output value by more than a threshold amount, automatically adjusting the optical stimulation again.

In at least some aspects, the corrective action includes halting the optical stimulation. In at least some aspects, the processor is further configured for, when the measurement deviates from the expected light output value by more than the threshold amount, receiving, from the light monitor, a second measurement of light generated by the light source to confirm the deviation when the second measurement also deviates from the expected light output value by more than the threshold amount.

In at least some aspects, automatically initiating a verification or measurement of a light output value includes periodically, automatically initiating the verification or measurement of the light output value. In at least some aspects, periodically, automatically initiating includes repeatedly automatically initiating the verification or measurement of the light output value at a regular predefined period. In at least some aspects, periodically, automatically initiating includes repeatedly automatically initiating the verification or measurement of the light output value according to a predefined pattern.

In another aspect, a non-transitory processor readable storage media includes instructions for monitoring optical stimulation using an optical stimulation system including a light source, a light monitor, and an optical lead coupled to the light source, wherein execution of the instructions by one or more processor devices performs actions, including: automatically initiating a verification or measurement of a light output value; in response to the initiation, receiving, from the light monitor, a measurement of light generated by the light source; and, when the measurement deviates from an expected light output value by more than a threshold amount, performing at least one of the following: sending a warning; or taking a corrective action.

In a further aspect, a method of monitoring optical stimulation using an optical stimulation system including a light source, a light monitor, and an optical lead coupled to the light source, includes automatically initiating a verification or measurement of a light output value; in response to the initiation, receiving, from the light monitor, a measurement of light generated by the light source; and, when the measurement deviates from an expected light output value by more than a threshold amount, performing at least one of the following: sending a warning; or taking a corrective action.

In at least some aspects of the non-transitory processor readable storage media or the method, the actions or steps further include directing the light monitor to make the measurement. In at least some aspects of the non-transitory processor readable storage media or the method, the actions or steps further include directing the light source to generate light that is expected to be at the expected light output level at a site where light is collected for measurement by the light monitor.

In at least some aspects of the non-transitory processor readable storage media or the method, sending a warning includes causing the control module to emit a vibratory or auditory warning. In at least some aspects of the non-transitory processor readable storage media or the method, the corrective action includes prompting or directing a user to adjust the optical stimulation. In at least some aspects of the non-transitory processor readable storage media or the method, the corrective action further includes, after the user adjusts the optical stimulation, receiving, from the light monitor, a second measurement of light generated by the light source; and, when the second measurement deviates from an expected light output value by more than a threshold amount, prompting or directing the user to adjust the optical stimulation.

In at least some aspects of the non-transitory processor readable storage media or the method, the corrective action includes automatically adjusting the optical stimulation. In at least some aspects of the non-transitory processor readable storage media or the method, the corrective action further includes, after automatically adjusting the optical stimulation, receiving, from the light monitor, a second measurement of light generated by the light source; and, when the second measurement deviates from an expected light output value by more than a threshold amount, automatically adjusting the optical stimulation again.

In at least some aspects of the non-transitory processor readable storage media or the method, the corrective action includes halting the optical stimulation. In at least some aspects of the non-transitory processor readable storage media or the method, the actions or steps further include, when the measurement deviates from the expected light output value by more than the threshold amount, receiving, from the light monitor, a second measurement of light generated by the light source to confirm the deviation when the second measurement also deviates from the expected light output value by more than the threshold amount.

In at least some aspects of the non-transitory processor readable storage media or the method, automatically initiating a verification or measurement of a light output value includes periodically, automatically initiating the verification or measurement of the light output value. In at least some aspects of the non-transitory processor readable storage media or the method, periodically, automatically initiating includes repeatedly automatically initiating the verification or measurement of the light output value at a regular predefined period. In at least some aspects of the non-transitory processor readable storage media or the method, periodically, automatically initiating includes repeatedly automatically initiating the verification or measurement of the light output value according to a predefined pattern. The invention is defined in the claims. Other embodiments, aspects, methods etc. are provided for illustrative purposes exclusively.

### BRIEF DESCRIPTION OF THE DRAWINGS

FIG. 1 is a schematic overview of one embodiment of components of an optical or optical/electrical stimulation system, including an electronic subassembly;
FIG. 2 is a schematic side view of one embodiment of an arrangement including a light source, an optional light monitor, an optical lead, and a connector lead;
FIG. 3 is a schematic cross-sectional view of one embodiment of the optical lead of FIG. 2;
FIG. 4A is a schematic side view of one embodiment of a control module configured to electrically couple to a lead or lead extension;
FIG. 4B is a schematic side view of one embodiment of a lead extension configured to electrically couple a lead to the control module of FIG. 4A;
FIG. 5 is a schematic side view of one embodiment of an electrical stimulation system that includes an electrical stimulation lead electrically coupled to a control module;
FIG. 6 is a schematic side view of one embodiment of an optical/electrical stimulation system with an optical/electrical stimulation lead coupled to a control module having a light source;
FIG. 7 is a schematic overview of one embodiment of components of a programming unit for an optical or optical/electrical stimulation system;
FIG. 8 is a flowchart for one embodiment of a method of monitoring optical stimulation;
FIG. 9 is a flowchart for another embodiment of a method of monitoring optical stimulation;
FIG. 10 is a flowchart for one embodiment of a method of prompting or directing a user to adjust stimulation parameters;
FIG. 11 is a flowchart for one embodiment of a method of automatically adjusting stimulation parameters; and
FIG. 12 is a diagram of one embodiment of a user interface for monitoring light output for optical stimulation.

### DETAILED DESCRIPTION

The present disclosure is directed to the area of implantable optical stimulation systems.

In some embodiments, the implantable optical stimulation system only provides optical stimulation. In other embodiments, the stimulation system can include both optical and electrical stimulation. In at least some of these embodiments, the optical stimulation system can be a modification of an electrical stimulation system to also, or instead, provide optical stimulation. Optical stimulation may include, but is not necessarily limited to, stimulation resulting from response to particular wavelengths or wavelength ranges of light or from thermal effects generated using light or any combination thereof.

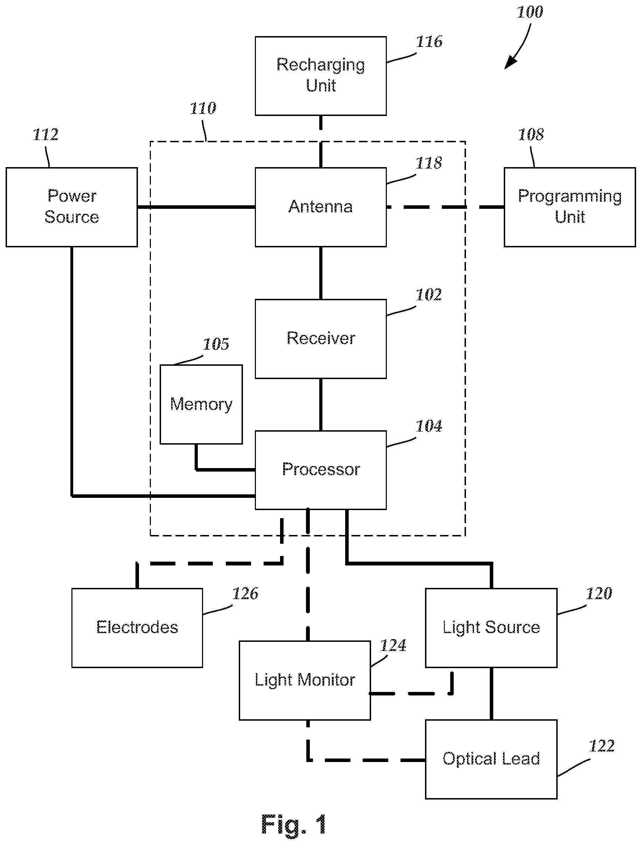

Figure 1 is a schematic overview of one embodiment of components of an optical stimulation system 100 (or combination optical/electrical stimulation system) including an electronic subassembly 110 disposed within implantable control module. In at least some embodiments, the optical stimulation system may also be capable of providing electrical stimulation through optional electrodes 126.

In at least some embodiments, selected components (for example, a power source 112, an antenna 118, a receiver 102, a processor 104, and a memory 105) of the optical stimulation system can be positioned on one or more circuit boards or similar carriers within a sealed housing of a control module. Any suitable processor 104 can be used and can be as simple as an electronic device that, for example, produces signals to direct or generate optical stimulation at a regular interval or the processor can be capable of receiving and interpreting instructions from an external programming unit 108 that, for example, allows modification of stimulation parameters or characteristics.

The processor 104 is generally included to control the timing and other characteristics of the optical stimulation system. For example, the processor 104 can, if desired, control one or more of the timing, pulse frequency, amplitude, and duration of the optical stimulation. In addition, the processor 104 can select one or more of the optional electrodes 126 to provide electrical stimulation, if desired. In some embodiments, the processor 104 selects which of the optional electrode(s) are cathodes and which electrode(s) are anodes.

Any suitable memory 105 can be used. The memory 105 illustrates a type of computer-readable media, namely computer-readable storage media. Computer-readable storage media may include, but is not limited to, nonvolatile, non-transitory, removable, and non-removable media implemented in any method or technology for storage of information, such as computer readable instructions, data structures, program modules, or other data. Examples of computer-readable storage media include RAM, ROM, EEPROM, flash memory, or other memory technology, magnetic storage devices, or any other medium which can be used to store the desired information and which can be accessed by a processor.

The processor 104 is coupled to a light source 120. Any suitable light source can be used including, but not limited to, light emitting diodes (LEDs), organic light emitting diodes (OLEDs), laser diodes, lamps, light bulbs, or the like or any combination thereof. In at least some embodiments, the optical stimulation system may include multiple light sources. In at least some embodiments, each of the multiple light sources may emit light having a different wavelength or different wavelength range. Any suitable wavelength or wavelength range can be used including, but not limited to, visible, near infrared, and ultraviolet wavelengths or wavelength ranges. In at least some embodiments, the optical stimulation system includes a light source that emits in the orange, red, or infrared wavelength ranges (for example, in the range of 600 to 1200 nm or in the range of 600 to 700 nm or in the range of 610 to 650 nm or 620 nm or the like.) In at least some embodiments, the optical stimulation system includes a light source that emits in the green or blue wavelength ranges (for example, in the range of 450 to 550 nm or in the range of 495 to 545 nm or the like.) A wavelength or wavelength range of a light source may be selected to obtain a specific therapeutic, chemical, or biological effect.

As described below, the light source 120 may be disposed within the control module or disposed external to the control module such as, for example, in a separate unit or module or as part of an optical lead. The processor 104 provides electrical signals to operate the light source 120 including, for example, directing or driving the generation of light by the light source, pulsing the light source, or the like. For example, the processor 104 can direct current from the power source 112 to operate the light source 120. In at least some embodiments, the light source 120 is coupled to one or more optical waveguides (such as an optical fiber or other optical transmission media) disposed in an optical lead 122. In at least some embodiments, the optical lead 122 is arranged so that one or more of the optical waveguides emits light from the distal portion of the optical lead (for example, the distal end or at one or more positions along the distal portion of the lead or any combination thereof).

Optionally, the processor 104 is also coupled to a light monitor 124 that is used to monitor or measure light from the light source 122. For example, the light monitor 124 can produce electrical or other signals in response to the light received by the light monitor. Any suitable light monitor 124 can be used including, but not limited to, photodiodes, phototransistors, photomultipliers, charge coupled devices (CCDs), light dependent resistors (LRDs), photo-emissive cells, photo-conductive ells, photo-voltaic cells, photo-junction devices, or the like or any combination thereof. The light monitor 124 may be used to measure or monitor the light emitted by the light source 120 or from the optical waveguide(s) (or other optical transmission media) of the optical lead 122. In at least some embodiments, the light monitor 124 may be coupled to one or more optical waveguides (or other optical transmission media) of the optical lead 122 to transmit the light along an optical lead for measurement or monitoring.

Any power source 112 can be used including, for example, a battery such as a primary battery or a rechargeable battery. Examples of other power sources include super capacitors, nuclear or atomic batteries, fuel cells, mechanical resonators, infrared collectors, flexural powered energy sources, thermally-powered energy sources, bioenergy power sources, bioelectric cells, osmotic pressure pumps, and the like. As another alternative, power can be supplied by an external power source through inductive coupling via an antenna 118 or a secondary antenna. The external power source can be in a device that is mounted on the skin of the user or in a unit that is provided near the user on a permanent or periodic basis. In at least some embodiments, if the power source 112 is a rechargeable battery, the battery may be recharged using the antenna 118 and a recharging unit 116. In some embodiments, power can be provided to the battery for recharging by inductively coupling the battery to the external recharging unit 116.

In at least some embodiments, the processor 104 is coupled to a receiver 102 which, in turn, is coupled to an antenna 118. This allows the processor 104 to receive instructions from an external source, such as programming unit 108, to, for example, direct the stimulation parameters and characteristics. The signals sent to the processor 104 via the antenna 118 and the receiver 102 can be used to modify or otherwise direct the operation of the optical stimulation system. For example, the signals may be used to modify the stimulation characteristics of the optical stimulation system such as modifying one or more of stimulation duration and stimulation amplitude. The signals may also direct the optical stimulation system 100 to cease operation, to start operation, to start charging the battery, or to stop charging the battery. In other embodiments, the stimulation system does not include the antenna 118 or receiver 102 and the processor 104 operates as initially programmed.

In at least some embodiments, the antenna 118 is capable of receiving signals (e.g., RF signals) from an external programming unit 108 (such as a clinician programmer or patient remote control or any other device) which can be programmed by a user, a clinician, or other individual. The programming unit 108 can be any unit that can provide information or instructions to the optical stimulation system 100. In at least some embodiments, the programming unit 108 can provide signals or information to the processor 104 via a wireless or wired connection. One example of a suitable programming unit is a clinician programmer or other computer operated by a clinician or other user to select, set, or program operational parameters for the stimulation. Another example of the programming unit 108 is a remote control such as, for example, a device that is worn on the skin of the user or can be carried by the user and can have a form similar to a pager, cellular phone, or remote control, if desired. In at least some embodiments, a remote control used by a patient may have fewer options or capabilities for altering stimulation parameters than a clinician programmer.

Optionally, the optical stimulation system 100 may include a transmitter (not shown) coupled to the processor 104 and the antenna 118 for transmitting signals back to the programming unit 108 or another unit capable of receiving the signals. For example, the optical stimulation system 100 may transmit signals indicating whether the optical stimulation system 100 is operating properly or not or indicating when the battery needs to be charged or the level of charge remaining in the battery. The processor 104 may also be capable of transmitting information about the stimulation characteristics so that a user or clinician can determine or verify the characteristics.

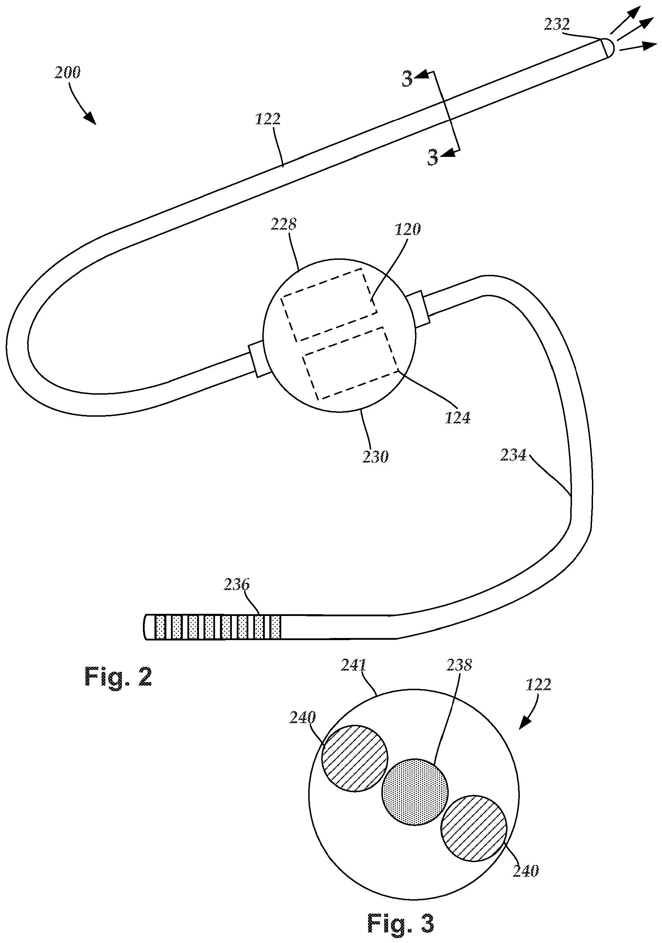

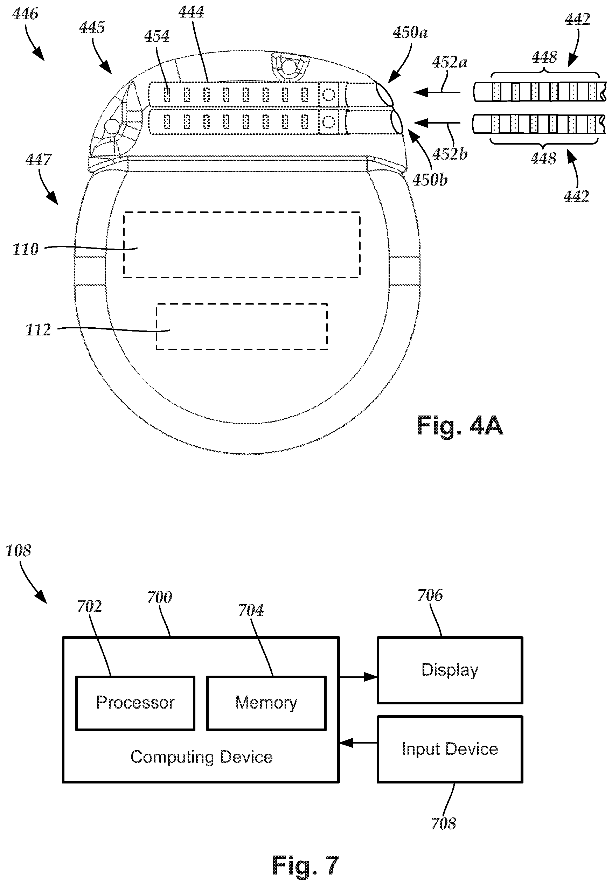

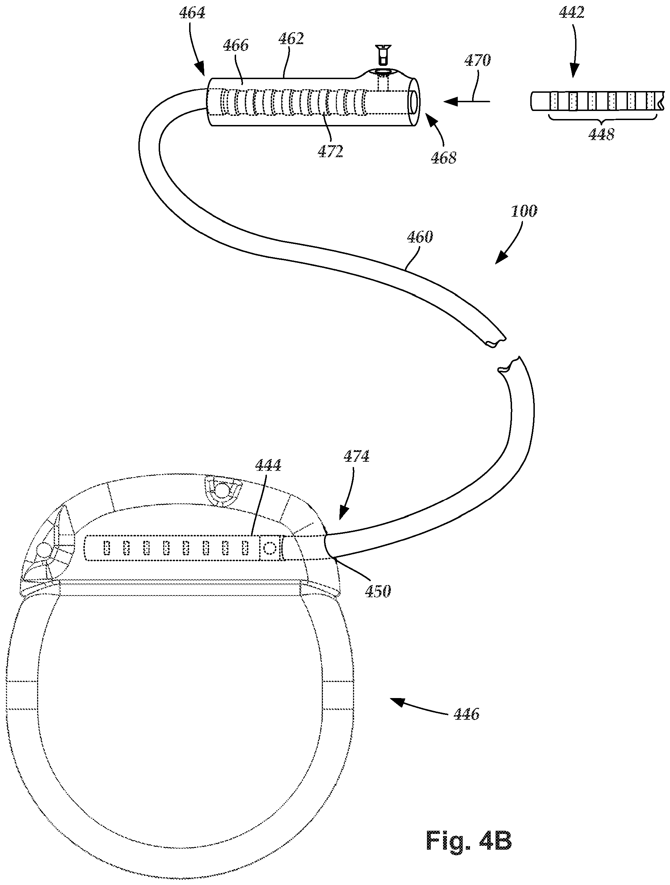

Figure 2 illustrates one embodiment of an arrangement 200 for an optical stimulation system that can be used with a control module (see, Figure 4). In at least some embodiments, the control module may be originally designed for use with an electrical stimulation system and adapted for use as an optical stimulation system via the arrangement 200.

The arrangement 200 includes a base unit 228 a light source 120 disposed in a housing 230, an optical lead 122 with one or more emission regions 232a, 232b of a distal portion from which light is emitted, and a connector lead 234 with one or more terminals 236 for coupling to a control module or lead extension, as described below. The optical lead 122 and connector lead 234, independently, may be permanently, or removably, coupled to the base unit 228. If removably coupleable to the base unit 228, the optical lead 122, connector lead 234, or both will have corresponding arrangements (for example, terminals and contacts) for transmission of light (for the optical lead) or electrical signals (for the connector lead) to the base unit 228. The one or more emission regions 232a, 232b may include a tip emission region 232a that emits distally away from the lead or may include a side emission regions 232b that emit at the sides of the lead or any combination thereof.

In addition to the light source 120, the base unit 228 can optionally include a light monitor 124. The base unit 228 may also include components such as electrical components associated with the light source 120 or light monitor 124, a heat sink, optical components (for example, a lens, polarizer, filter, or the like), a light shield to reduce or prevent light emission out of the housing of the base unit or to reduce or prevent extraneous light from penetrating to the light monitor 124 or the like. The housing 230 of the base unit 228 can be made of any suitable material including, but not limited to, plastic, metal, ceramic, or the like, or any combination thereof. As the base unit 228 is to be implanted, the housing 230 is preferably made of a biocompatible material such as, for example, silicone, polyurethane, titanium or titanium alloy, or any combination thereof.

In at least some embodiments, the optical lead 122, as illustrated in cross-section in Figured 3, includes a lead body 241 and one or more optical waveguides 238 (or other optical transmission media) for transmission of light from the light source 120 with emission along the one or more emission regions 232a, 232b disposed on the distal portion of the optical lead. In the illustrated embodiment, the light is emitted at the distal tip of the lead. In other embodiments, the light may be emitted at one or more points along the length of at least the distal portion of the lead. In some embodiments with multiple light sources, there may be separate optical waveguides for each light source or light from multiple light sources may be transmitted along the same optical waveguide(s). The optical lead 122 may also include one or more optical components, such as a lens, diffuser, polarizer, filter, or the like, at the distal portion of the lead (for example, at the terminal end of the optical waveguide 238) to modify the light transmitted through the optical waveguide.

In at least some embodiments that include a light monitor 124, the optical lead 122 may include one or more optical waveguides 240 (or other optical transmission media) that receive light emitted from the light source 120 and transmitted by the optical waveguide 238 in order to measure or monitor the light emitted at the one or more emission regions 232a, 232b of the optical lead. The optical waveguide(s) 240 transmit light from the one or more emission regions 232a, 232b of the optical lead to the light monitor 124 in the base unit 228. The optical lead 122 may also include one or more optical components, such as a lens, diffuser, polarizer, filter, or the like, at the distal portion of the lead (for example, at the terminal end of the optical waveguide 240) to modify the light received by the optical waveguide(s) 240.

The connector lead 234 includes conductors (e.g., wires - not shown) disposed in a lead body extending along the connector lead 234 to the terminals 236 on the proximal end of the connector lead. As an alternative, the connector lead 234 may be permanently attached to a control module or other device where the conductors then attach to contact points within the control module or other device. The conductors carry electrical signals to the base unit 228 and the light source 120 and, optionally, other electrical components in the base unit for operation of the light source 120. The conductors may also carry electrical signals from the optional light monitor 124 in the base unit 228 to the control module or other device. These electrical signals may be generated by the light monitor 124 in response to light received by the light monitor.

Figure 4A is a schematic side view of one embodiment of proximal ends 442 of one or more leads (for example, connector lead 234 of Figure 2) or lead extensions 460 (see, Figure 4B) coupling to a control module 446 (or other device) through one or more control module connectors 444. The one or more proximal ends 442 include terminals 448 (for example, terminals 236 of connector lead 234).

The control module connector 444 defines at least one port 450a, 450b into which a proximal end 442 can be inserted, as shown by directional arrows 452a and 452b. The control module 446 (or other device) can define any suitable number of ports including, for example, one, two, three, four, five, six, seven, eight, or more ports.

The control module connector 444 also includes a plurality of connector contacts, such as connector contact 454, disposed within each port 450a and 450b. When the proximal end 442 is inserted into the ports 450a and 450b, the connector contacts 454 can be aligned with a plurality of terminals 448 disposed along the proximal end(s) 442. Examples of connectors in control modules are found in, for example, U.S. Patent No. 7,244,150 and 8,224,450,

The control module 446 typically includes a connector housing 445 and a sealed electronics housing 447. An electronic subassembly 110 (see, Figure 1) and an optional power source 112 (see, Figure 1) are disposed in the electronics housing 447.

Figure 4B is a schematic side view of a portion of another embodiment of an optical stimulation system 100. The optical stimulation system 100 includes a lead extension 460 that is configured to couple one or more proximal ends 442 of a lead to the control module 446. In Figure 4B, the lead extension 460 is shown coupled to a single port 450 defined in the control module connector 444. Additionally, the lead extension 460 is shown configured to couple to a single proximal end 442 of a lead (for example, the connector lead 234 of Figure 2).

A lead extension connector 462 is disposed on the lead extension 460. In Figure 4B, the lead extension connector 462 is shown disposed at a distal end 464 of the lead extension 460. The lead extension connector 462 includes a connector housing 466. The connector housing 466 defines at least one port 468 into which terminals 448 of the proximal end 442 of the lead can be inserted, as shown by directional arrow 470. The connector housing 466 also includes a plurality of connector contacts, such as connector contact 472. When the proximal end 442 is inserted into the port 468, the connector contacts 472 disposed in the connector housing 466 can be aligned with the terminals 448 for electrical coupling.

In at least some embodiments, the proximal end 474 of the lead extension 460 is similarly configured as a proximal end 442 of a lead. The lead extension 460 may include a plurality of electrically conductive wires (not shown) that electrically couple the connector contacts 472 to a proximal end 474 of the lead extension 460 that is opposite to the distal end 464. In at least some embodiments, the conductive wires disposed in the lead extension 460 can be electrically coupled to a plurality of terminals (not shown) disposed along the proximal end 474 of the lead extension 460. In at least some embodiments, the proximal end 474 of the lead extension 460 is configured for insertion into a connector disposed in another lead extension (or another intermediate device). In other embodiments (and as shown in Figure 4B), the proximal end 474 of the lead extension 460 is configured for insertion into the control module connector 144.

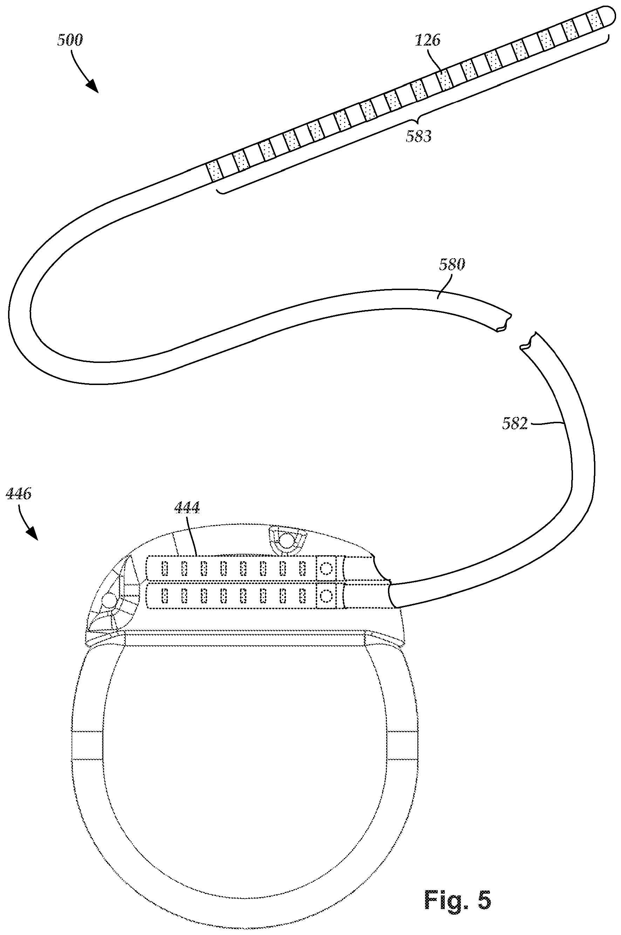

In some embodiments, the optical stimulation system may also be an electrical stimulation system. Figure 5 illustrates schematically one embodiment of an electrical stimulation system 500. The electrical stimulation system includes a control module 446 (e.g., a stimulator or pulse generator) and an electrical stimulation lead 580 coupleable to the control module 446. The same control module 446 can be utilized with the arrangement 200 (Figure 2) for optical stimulation and an electrical stimulation lead 580. With respect to the optical/electrical stimulation system of Figure 1, the control module 446 can include the electronic subassembly 110 (Figure 1) and power source 112 (Figure 1) and the electrical stimulation lead 580 can include the electrodes 126. The optical arrangement 200 of Figure 2 can be inserted into another port of the control module 446.

The lead 580 includes one or more lead bodies 582, an array of electrodes 583, such as electrode 126, and an array of terminals (e.g., 448 in Figure 4A-4B) disposed along the one or more lead bodies 582. In at least some embodiments, the lead is isodiametric along a longitudinal length of the lead body 582. Electrically conductive wires, cables, or the like (not shown) extend from the terminals to the electrodes 126. Typically, one or more electrodes 126 are electrically coupled to each terminal. In at least some embodiments, each terminal is only connected to one electrode 126.

The lead 580 can be coupled to the control module 446 in any suitable manner. In at least some embodiments, the lead 580 couples directly to the control module 446. In at least some other embodiments, the lead 580 couples to the control module 446 via one or more intermediate devices. For example, in at least some embodiments one or more lead extensions 460 (*see e.g*., Figure 4B) can be disposed between the lead 580 and the control module 446 to extend the distance between the lead 580 and the control module 446. Other intermediate devices may be used in addition to, or in lieu of, one or more lead extensions including, for example, a splitter, an adaptor, or the like or combinations thereof. It will be understood that, in the case where the electrical stimulation system 500 includes multiple elongated devices disposed between the lead 580 and the control module 446, the intermediate devices may be configured into any suitable arrangement.

The electrical stimulation system or components of the electrical stimulation system, including one or more of the lead bodies 582 and the control module 446, are typically implanted into the body of a patient. The electrical stimulation system can be used for a variety of applications including, but not limited to, brain stimulation, neural stimulation, spinal cord stimulation, muscle stimulation, and the like.

The electrodes 126 can be formed using any conductive, biocompatible material. Examples of suitable materials include metals, alloys, conductive polymers, conductive carbon, and the like, as well as combinations thereof. In at least some embodiments, one or more of the electrodes 126 are formed from one or more of: platinum, platinum iridium, palladium, palladium rhodium, or titanium. The number of electrodes 126 in each array 583 may vary. For example, there can be two, four, six, eight, ten, twelve, fourteen, sixteen, or more electrodes 126. As will be recognized, other numbers of electrodes 126 may also be used.

Examples of electrical stimulation systems with leads are found in, for example, U.S. Patents Nos. 6,181,969; 6,295,944; 6,391,985; 6,516,227; 6,609,029; 6,609,032; 6,741,892; 7,244,150; 7,450,997; 7,672,734;7,761,165; 7,783,359; 7,792,590; 7,809,446; 7,949,395; 7,974,706; 8,831,742; 8,688,235; 6,175,710; 6,224,450; 6,271,094; 6,295,944; 6,364,278; and 6,391,985; U.S. Patent Applications Publication Nos. 2007/0150036; 2009/0187222; 2009/0276021; 2010/0076535; 2010/0268298; 2011/0004267; 2011/0078900; 2011/0130817; 2011/0130818; 2011/0238129; 2011/0313500; 2012/0016378; 2012/0046710; 2012/0071949; 2012/0165911; 2012/0197375; 2012/0203316; 2012/0203320; 2012/0203321; 2012/0316615; 2013/0105071; 2011/0005069; 2010/0268298; 2011/0130817; 2011/0130818; 2011/0078900; 2011/0238129; 2011/0313500; 2012/0016378; 2012/0046710; 2012/0165911; 2012/0197375; 2012/0203316; 2012/0203320; and 2012/0203321,

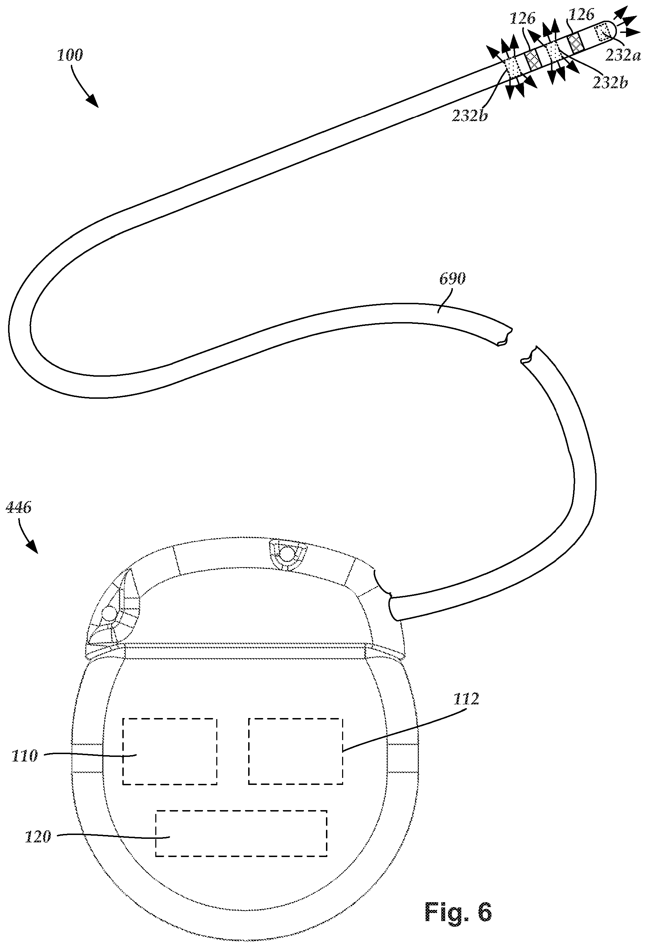

Figure 6 illustrates other optional embodiments. For example, Figure 6 illustrates one embodiment of an optical/electrical stimulation system 100 with a lead 690 with both electrodes 126 and an optical waveguide that emits light from the from one more emission regions 232a, 232b of the lead. In some embodiments, the lead 690 can be coupled to the base unit 228 and connector lead 234 of Figure 2 with conductors (and optionally connector contacts if the lead 690 or connector lead 234 are removable from the base unit 228) electrically coupling the terminals 236 of the connector lead to the electrodes 126 of the lead 690.

Figure 6 also illustrates one embodiment of a control module 446 that also includes a light source 120 within the control module. Such an arrangement can replace the base unit 228 and connector lead 234 of Figure 2 and may include a lead extension 460.

Figure 7 illustrates one embodiment of a programming unit 108. The programming unit 108 can include a computing device 700 or any other similar device that includes a processor 702 and a memory 704, a display 706, and an input device 708.

The computing device 700 can be a computer, tablet, mobile device, or any other suitable device for processing information or programming an optical stimulation system. The computing device 700 can be local to the user or can include components that are non-local to the computer including one or both of the processor 702 or memory 704 (or portions thereof). For example, in at least some embodiments, the user may operate a terminal that is connected to a non-local computing device. In other embodiments, the memory can be non-local to the user.

The computing device 700 can utilize any suitable processor 702 including at least one hardware processors that may be local to the user or non-local to the user or other components of the computing device. The processor 702 is configured to execute instructions provided to the processor 702, as described below.

Any suitable memory 704 can be used for the computing device 702. The memory 704 illustrates a type of computer-readable media, namely computer-readable storage media. Computer-readable storage media may include, but is not limited to, nonvolatile, non-transitory, removable, and non-removable media implemented in any method or technology for storage of information, such as computer readable instructions, data structures, program modules, or other data. Examples of computer-readable storage media include RAM, ROM, EEPROM, flash memory, or other memory technology, CD-ROM, digital versatile disks ("DVD") or other optical storage, magnetic cassettes, magnetic tape, magnetic disk storage or other magnetic storage devices, or any other medium which can be used to store the desired information and which can be accessed by a computing device.

Communication methods provide another type of computer readable media; namely communication media. Communication media typically embodies computer-readable instructions, data structures, program modules, or other data in a modulated data signal such as a carrier wave, data signal, or other transport mechanism and include any information delivery media. The terms "modulated data signal," and "carrier-wave signal" includes a signal that has at least one of its characteristics set or changed in such a manner as to encode information, instructions, data, and the like, in the signal. By way of example, communication media includes wired media such as twisted pair, coaxial cable, fiber optics, wave guides, and other wired media and wireless media such as acoustic, RF, infrared, and other wireless media.

The display 706 can be any suitable display device, such as a monitor, screen, display, or the like, and can include a printer. In at least some embodiments, the display 706 may form a single unit with the computing device 700. The input device 708 can be, for example, a keyboard, mouse, touch screen, track ball, joystick, voice recognition system, or any combination thereof, or the like.

The methods and systems described herein may be embodied in many different forms and should not be construed as limited to the embodiments set forth herein. Accordingly, the methods and systems described herein may take the form of an entirely hardware embodiment, an entirely software embodiment or an embodiment combining software and hardware aspects. Systems mentioned herein typically include memory and typically include methods for communication with other devices including mobile devices. Methods of communication can include both wired and wireless (for example, RF, optical, or infrared) communications methods and such methods provide another type of computer readable media; namely communication media. Wired communication can include communication over a twisted pair, coaxial cable, fiber optics, wave guides, or the like, or any combination thereof. Wireless communication can include RF, infrared, acoustic, near field communication, Bluetooth^{™}, or the like, or any combination thereof.

This optical power output by the light source 120 is often different from the optical power output at the distal end of the optical lead. For example, the components in, along, or mounted on the light source 120 or optical lead 122, as well as the manufacturing process, can produce light loss to reduce the optical power output at the distal end of the optical lead. Moreover, the individual light sources and optional light monitors, as well as other optical components such as the optical waveguides, generally have variations of performance from part to part. In addition, the power source 112 can have an associated tolerance and can vary between individual units.

In at least some instances, optical stimulation is typically not felt by the patient, but the effectiveness of the optical stimulation therapy often results from the long-term application of the therapy. Failure to produce the programmed optical stimulation therapy may reduce or eliminate the efficacy of the therapy. In at least some instances, the optical stimulation system may register that the control module is directing the light source to produce the optical stimulation therapy, but a failure, damage, or other defect within the optical components (for example, the light source or optical lead) or other components may result in reduction in, or complete loss of, effectiveness of the optical stimulation therapy. The patient or clinician may be unaware of the failure or the reduction or loss of effectiveness of the therapy.

In many instances, it is desirable to have a mechanism for the optical stimulation system to confirm that the system is delivering the optical stimulation and, preferably, at the selected light output value (measured, for example, at the distal end of the optical lead or at the light source). Accordingly, it is useful for the system to be configured to conduct periodic automated checks to verify that the optical stimulation system is still providing the optical stimulation at the selected output level. In at least some embodiments, the optical stimulation system can relay information to the patient or the clinician (or both) if an anomaly is detected. In at least some embodiments, the optical stimulation system can attempt to adjust the stimulation parameters to attempt to return the optical stimulation to the selected output level.

In at least some embodiments, the processor 104 periodically directs the light monitor 124 to measure the light output value at the distal end of the optical lead 122 (or alternatively emitted by the light source 120). The period may be regular or irregular (for example, randomly or pseudorandomly selected within a range of time). Examples of a period of time between measurements include, but are not limited to, 5, 10, 15, 30, 60, 90, or more minutes; 2, 4, 6, 8, 12, or more hours; 1, 2, 3, 4, 5, 7, or more days. In at least some embodiments, the period of time between measurements is selectable or programmable by a user, such as a clinician or programmer. In at least some embodiments, a schedule of measurements, or whether the measurements are performed randomly or pseudorandomly within a range of time (as well as the range of time), is selectable or programmable by a user, such as a clinician or programmer.

In at least some embodiments, the optical stimulation system may store measurement of light output values to provide historical measurement data. For example, such measurements may be stored in a memory 105 of a control module 446 or memory 704 of a programming unit 108. In at least some embodiments, the stored measurements may be used for troubleshooting or analysis of the system's light output.

The processor 104 evaluates the resulting measurement to determine whether the optical stimulation system is producing the programmed light therapy. In at least some embodiments, the processor may convert measurements of the light monitor in mA or mV (or other suitable units) to light output values in mW (or other suitable units.) As an example, the processor 104 may compare the measurement and an expected light output value and determine whether the measurement deviates from the expected light output value by more than a threshold amount. In at least some embodiments, the threshold amount may be programmed or otherwise selected by a user, such as a clinician or programmer. The threshold amount may be a numerical value or a percentage or any other suitable parameter that represents an acceptable distance from the expected light output value.

In at least some embodiments, the expected light output value may be programmed or otherwise selected by a user, such as a clinician or programmer. In at least some embodiments, the expected light output value may be a measured light output value (or average of measured light output values) that is established as a baseline during or following, for example, programming of the optical stimulation system. In at least some embodiments, the expected light output value may be determined using a calibration table or formula. Examples of generating and using calibration tables and formulas are found in U.S. Provisional Patent Application Serial No. 62/647,561, entitled "Optical Stimulation Systems with Calibration and Methods of Making and Using"

In at least some embodiments, the processor may convert measurements of the light monitor in mA or mV (or other suitable units) to light output values in mW (or other suitable units.) A calibration table or calibration formula, as described above, may be used for this conversion. Alternatively, any other mechanism for conversion can be used.

In some embodiments, the processor 104 may direct multiple measurements over a period of time (for example, over 1, 5, 10, 15, 30, 45, or 60 minutes or more) to confirm the deviation. In some embodiments, the processor 104 may confirm the deviation after a specified number (for example, two, three, four, or more) of the periodic measurements exceed the threshold. In some of these embodiments, the deviation is confirmed if those measurement that exceed the threshold are consecutive. In other embodiments, the measurements may not be required to be consecutive, but rather are made with a predetermined time period (for example, in 6, 8, or 12 hours or 1, 2, or 7 days) or are a predetermined number or percentage of the measurements (for example, 3 out of 4 consecutive measurements or 75% of the consecutive measurements.) In at least some embodiments, the number of measurements that confirm a deviation is selectable or programmable by a user, such as a clinician or programmer. In at least some embodiments, whether the measurements must be consecutive or not and, if not, what criteria confirms a deviation, may be selectable or programmable by a user, such as a clinician or programmer.

In at least some embodiments, one or more of the threshold amount, the period of time between measurements, the measurement schedule, the number of measurement to confirm a deviation, whether the measurements must be consecutive or not, or any of the other settings described herein or any combination of these settings may be password protected to prevent or hinder changing these settings by individuals other than an authorized person such as a clinician. In at least some embodiments, the optical stimulation system may include a user interface (for example, as part of a programming unit 108) to set, adjust, change, or modify one or more of these settings.

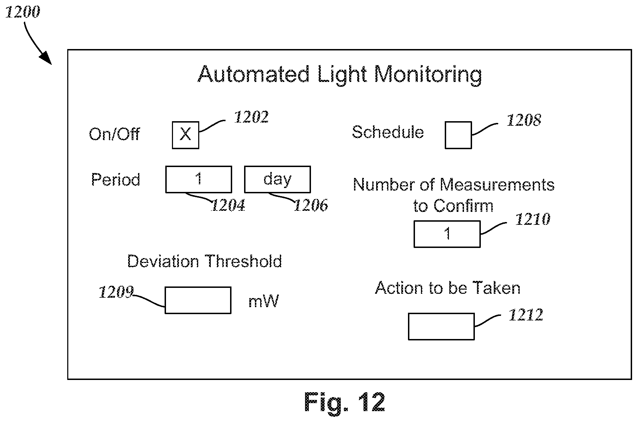

Figure 12 illustrates one embodiment of a user interface 1200 for inputting settings for automated light monitoring. The illustrated embodiment includes a control 1202 for turning the automated light monitoring on or off, an input box for the value of the period of time 1204 between measurement, an input box for the units 1206 for the period of time, a control 1208 for, instead, setting a measurement schedule, an input box 1209 for the threshold for allowed deviation from the expected light output value, an input box 1210 for indicating the number of measurements needed to confirm a deviation, and a control 1212 for selecting or defining the type of warning or corrective action that is taken when a deviation is measured or confirmed. It will be understood that many other interface designs are possible and that the controls for monitoring light output can be integrated into a programming or other user interface. For example, the user interface may define a periodic measurement by the number of measurements in a particular period of time such as, for example, the number of measurement per day.

In at least some embodiments, if a deviation is detected or confirmed, an audible, visual, vibratory, or other warning is sent to the patient. For example, the control module may emit an audible or vibratory warning. As another example, a remote control or recharging unit used by the patient may present an audible, visual, vibratory, or other warning which is received through communication with the control module. For example, the control module, programming unit, remote control, recharging unit, or other device used to make the request may include a buzzer or speaker for providing an audible warning. In some embodiments, the warning may direct the patient to contact or visit the clinician.

In some embodiments, if a deviation is detected or confirmed, an audible, visual, vibratory, or other warning is sent to the clinician. For example, the control module may communicate to a remote control or recharging unit used by the patient that may send the warning to the clinician over the Internet, over a mobile network, or through other wired or wireless communication.

In at least some embodiments, the optical stimulation system can provide an indication (for example, through a patient's remote control) to the patient or clinician to recommend adjustment to the therapy. For example, the optical stimulation system may direct the patient or clinician to adjust one or more stimulation parameters (for example, the amount of light generated by the light source or the signal sent to the light source) and may propose amount for the adjustment.

In at least some embodiments, the optical stimulation system may automatically adjust the therapy by, for example, adjusting one or more stimulation parameters such as, (for example, the amount of light generated by the light source or the signal sent to the light source.

In at least some embodiments, if the patient or clinician is directed to adjust the therapy or the system automatically adjusts the therapy, the system may obtain further measurements of the light output value using the light monitor 124 to observe the results of the adjustments. The system may iteratively direct the patient or clinician to adjust the therapy or automatically adjust the therapy and then obtain measurements to observe the results. In at least some embodiments, if adjustments to the therapy are ineffective or result in unacceptable light output levels (for example, levels that are too high or too low), the system may take one or more corrective actions such as, for example, operating the system using a set of stimulation parameters that are selected to produce a safe level of stimulation or limit the light output level, halt the stimulation, or send a warning to the patient or clinician or both, or any combination thereof.

In at least some embodiments, the measurements from the light monitor 124 are provided to the processor 104 of a control module 446 or the processor 702 of a programming unit 108 or a processor of another device. The processor includes an algorithm or other computer program that utilizes the measurements by the light monitor 124 and compares the measurement to expected light output values or other metrics to determine whether the desired optical stimulation is being delivered. In some instances, if the measurements indicate that the desired optical stimulation is not being delivered, the system may generate a warning, take a corrective action, or any combination thereof. For example, the processor may utilize the current stimulation parameters and, optionally, other information regarding the patient, disease or disorder, and the like to determine an adjustment to one or more of the stimulation parameters. The processor may communicate the adjustment to a clinician or other user or to the external programming unit 108 or control module 446.

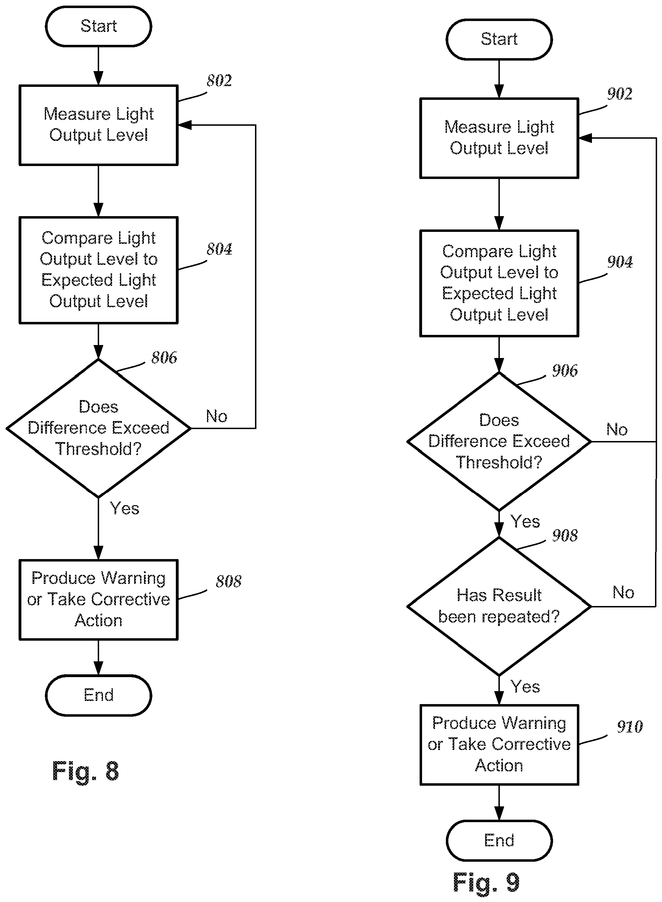

Figure 8 is a flowchart of one embodiment of a method of automatically monitoring optical stimulation. In step 802, a light output level is measured by the light monitor 124 (or any other suitable device). This can be performed periodically or when requested by the processor 104 or other device, such as a programming unit 108. In step 804, the measured light output level is compared to an expected light output level. In step 806, it is determined whether the difference between the measurement and the expected light level exceeds a threshold. If not, the cycle of measurements and comparisons in steps 802 to 806 can occur repeatedly. If the difference exceeds the threshold, in step 808 the system can produce a warning, as described above, or take a corrective action, as described above, or any combination thereof. In at least some embodiments, steps 802 to 808 are repeated periodically or continuously.

Figure 9 is a flowchart of one embodiment of a method of automatically monitoring optical stimulation. In step 902, a light output level is measured by the light monitor 124 (or any other suitable device). This can be performed periodically or when requested by the processor 104 or other device, such as a programming unit 108. In step 904, the measured light output level is compared to an expected light output level. In step 906, it is determined whether the difference between the measurement and the expected light level exceeds a threshold. If not, the cycle of measurements and comparisons in steps 902 to 906 can occur repeatedly. If the difference exceeds the threshold, the system queries whether the threshold has been exceeded multiple times in step 908. As described above, the processor 104 may confirm the deviation after a specified number (for example, two, three, four, or more) of the periodic measurements exceed the threshold. In some of these embodiments, the deviation is confirmed if those measurement that exceed the threshold are consecutive. In other embodiments, the measurements may not be required to be consecutive, but rather are made with a predetermined time period (for example, in 6, 8, or 12 hours or 1, 2, or 7 days) or are a predetermined number or percentage of the measurements (for example, 3 out of 4 consecutive measurements or 75% of the consecutive measurements.) If the result of the query in step 908 is no, then the cycle of measurements, comparisons, and queries in steps 902 to 908 can occur repeatedly. If the result of the query in step 908 is yes, in step 910 the system can produce a warning, as described above, or take a corrective action, as described above, or any combination thereof. In at least some embodiments, steps 902 to 910 are repeated periodically or continuously.

In steps 808 and 910, the warning can be sent to the user (for example, through a control module, a remote control, a mobile phone, a tablet, a computer, or other device), to a clinician or care provider (for example, via a network to a programming unit, mobile phone, tablet, computer, or other device), or any combination thereof.

In steps 808 and 910, the corrective action can be one or more of prompting or directing the patient or clinician to adjust one or more of the stimulation parameters to select a different stimulation program, automatically adjusting one or more of the stimulation parameters, operating the system using a set of stimulation parameters or stimulation program that is selected to produce a safe level of stimulation, halt the stimulation, or the like or any combination thereof.

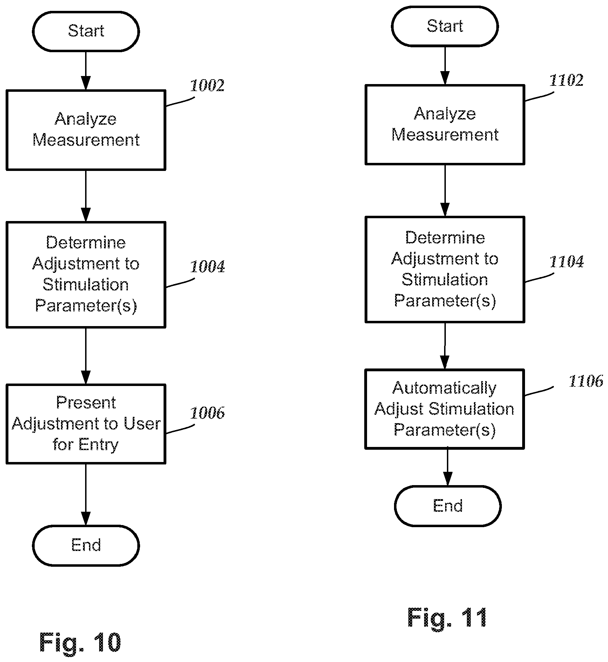

As indicated a corrective action may include prompting or directing adjustment to one or more stimulation parameters or automatically adjusting one or more stimulation parameters. Figure 10 is a flowchart of one embodiment of a method of prompting or directing adjustment to one or more stimulation parameters. In step 1002, the measurement by the light monitor 124 is analyzed. For example, the measurement may be analyzed to determine if the light output value is higher or lower in intensity than expected.

In step 1004, the processor determines an adjustment to one or more of the stimulation parameters in view of the analysis. Examples of stimulation parameters that can be adjusted include, but are not limited to, the amount of light generated by the light source, the expected light output level, the driving signal sent to the light source, light pulse or optical stimulation duration, light pulse patterns, other pulse timing parameters, and the like. The analysis and generation of the adjustment can be performed by the processor 104, external programming unit 108, control module 446, or any combination thereof. As an alternative to a specific adjustment to one or more stimulation parameters, the processor may select a predefined stimulation program.

In step 1006, the adjustment to the one or more stimulation parameters (or the selected predefined stimulation program) is presented to a user for entry. For example, the adjustment may be presented to a patient on a remote control, programming unit, mobile phone, tablet, or computer that is in communication with the control module. As another example, the adjustment may be presented to a clinician or other care giver on a programming unit, mobile phone, tablet, computer that is in communication with the control module. The device may direct or prompt the user to make the adjustment (or select the predefined stimulation program). If the user does not respond, the device optionally may send a warning to the patient, a clinician, a care giver, or any other suitable individual or device. In some embodiments, the system may automatically make the adjustment (or select the predefined stimulation program) if the user does not respond in a specified time period. In other embodiments, the system does not make the adjustment (or select the predefined stimulation program) automatically. In at least some embodiments, steps 1002 to 1006 are repeated periodically or continuously.

Figure 11 is a flowchart of one embodiment of a method of automatically adjusting one or more stimulation parameters. In step 1102, the measurement by the light monitor 124 is analyzed. For example, the measurement may be analyzed to determine if the light output value is higher or lower in intensity than expected.

In step 1104, the processor determines an adjustment to one or more of the stimulation parameters in view of the analysis. Examples of stimulation parameters that can be adjusted include, but are not limited to, the amount of light generated by the light source, the expected light output level, the driving signal sent to the light source, light pulse or optical stimulation duration, light pulse patterns, other pulse timing parameters, and the like. The analysis and generation of the adjustment can be performed by the processor 104, external programming unit 108, control module 446, or any combination thereof. As an alternative to a specific adjustment to one or more stimulation parameters, the processor may select a predefined stimulation program.

In step 1106, the system automatically makes the adjustment to the one or more stimulation parameters (or selects the predefined stimulation program). In at least some embodiments, the system sends a notice of the adjustment to the patient on a remote control, programming unit, mobile phone, tablet, or computer that is in communication with the control module or to a clinician or other care giver on a programming unit, mobile phone, tablet, computer that is in communication with the control module. In at least some embodiments, the system may permit the patient, clinician, or other care giver to return the system to the previous set of stimulation parameters or stimulation program. In at least some embodiments, steps 1102 to 1106 are repeated periodically or continuously.

In at least some embodiments of the methods illustrated in Figures 10 and 11, the system may utilize a step-wise methodology to altering, or prompting or directing alteration of, the stimulation parameters. For example, the system may alter, or prompt or direct alteration of, one or more stimulation parameters based on the light monitor measurements and then observe the results of the alteration as measured using the light monitor (or based on other input such as patient or clinician feedback.) In at least some embodiments, the system waits for a latency period to allow a clinical effect (therapeutic or side effect) to be noticeable to the patient, clinician, or other individual.

The processes illustrated in Figures 8-11 can be used as a feedback loop to adjust stimulation parameters. The feedback loop may be part of a programming session. Alternatively or additionally, the optical stimulation system may initiate the feedback loop on a regular or irregular basis or when requested by a user, clinician, or other individual to adjust stimulation parameters.

It will be understood that each block of the flowchart illustrations, and combinations of blocks in the flowchart illustrations and methods disclosed herein, can be implemented by computer program instructions. These program instructions may be provided to a processor to produce a machine, such that the instructions, which execute on the processor, create means for implementing the actions specified in the flowchart block or blocks disclosed herein. The computer program instructions may be executed by a processor to cause a series of operational steps to be performed by the processor to produce a computer implemented process. The computer program instructions may also cause at least some of the operational steps to be performed in parallel. Moreover, some of the steps may also be performed across more than one processor, such as might arise in a multi-processor computer system. In addition, at least one process may also be performed concurrently with other processes, or even in a different sequence than illustrated without departing from the scope of the invention.

The computer program instructions can be stored on any suitable computer-readable medium including, but not limited to, RAM, ROM, EEPROM, flash memory or other memory technology, CD-ROM, digital versatile disks ("DVD") or other optical storage, magnetic cassettes, magnetic tape, magnetic disk storage or other magnetic storage devices, or any other medium which can be used to store the desired information and which can be accessed by a computing device.

The invention is defined in the following claims:

## Claims

1. An optical stimulation system, comprising:
an implantable light source (120) configured to produce light for optical stimulation;
an implantable light monitor (124);
an implantable optical lead (122) coupled, or coupleable, to the light source and the light monitor; and
an implantable control module (446) coupled, or coupleable, to the light source and the light monitor, the control module comprising
a memory (105), and
a processor (104) coupled to the memory and configured for
automatically initiating a verification or measurement of a light output value;
in response to the initiation, receiving, from the light monitor, a measurement of light generated by the light source; and
when the measurement deviates from an expected light output value by more than a threshold amount, performing at least one of the following:
sending a warning; or
taking a corrective action.

2. The optical stimulation system of claim 1, wherein the processor is further configured for directing the light monitor to make the measurement.

3. The optical stimulation system of any one of claims 1 or 2, wherein the processor is further configured for directing the light source to generate light that is expected to be at the expected light output level at a site where light is collected for measurement by the light monitor.

4. The optical stimulation system of any one of claims 1-3, wherein the light monitor is configured to measure a light output level directly from the light source.

5. The optical stimulation system of any one of claims 1-4, wherein the optical lead further comprises a first optical waveguide (238) configured to receive light generated by the light source and emit the light from a distal portion of the optical lead for the optical stimulation and a second optical waveguide (240) configured to receive a portion of the light emitted from the distal portion of the optical lead and direct the received portion of the light to the light monitor, wherein the light monitor is configured to measure a light output level from the light emitted from the distal portion of the optical lead.

6. The optical stimulation system of any one of claims 1-5, further comprising an external device (108) configured for communication with the control module, wherein the processor is configured to send the warning to the external device and the external device is configured for, in response to receiving the warning, providing a visual or auditory message to a user.

7. The optical stimulation system of claim 6, wherein the external device is a programming unit, a clinician programmer, or a patient remote control.

8. The optical stimulation system of any one of claims 1-7, wherein sending a warning comprises causing the control module to emit a vibratory or auditory warning.

9. The optical stimulation system of any one of claims 1-8, wherein the corrective action comprises prompting or directing a user to adjust the optical stimulation if the measurement deviates by more than a threshold amount from an expected light output level.

10. The optical stimulation system of any one of claims 1-8, wherein the corrective action comprises automatically adjusting the optical stimulation if the measurement deviates by more than a threshold amount from an expected light output level.

11. The optical stimulation system of any one of claims 1-8, wherein the corrective action comprises halting the optical stimulation.

12. The optical stimulation system of any one of claims 1-11, wherein the processor is further configured for, when the measurement deviates from the expected light output value by more than the threshold amount, receiving, from the light monitor, a second measurement of light generated by the light source to confirm the deviation when the second measurement also deviates from the expected light output value by more than the threshold amount.

13. The optical stimulation system of any one of claims 1-12, wherein automatically initiating a verification or measurement of a light output value comprises periodically, automatically initiating the verification or measurement of the light output value.

14. A non-transitory processor readable storage media that includes instructions for monitoring optical stimulation using an optical stimulation system comprising an implantable light source (120), an implantable light monitor (124), and an implantable optical lead (122) coupled to the light source, wherein execution of the instructions by one or more processor devices performs actions, comprising:
automatically initiating a verification or measurement of a light output value;
in response to the initiation, receiving, from the light monitor, a measurement of light generated by the light source; and
when the measurement deviates from an expected light output value by more than a threshold amount, performing at least one of the following:
sending a warning; or
taking a corrective action.

15. A method of monitoring optical stimulation using an optical stimulation system comprising an implantable light source (120), an implantable light monitor (124), and an implantable optical lead (122) coupled to the light source, the method comprising:
automatically initiating a verification or measurement of a light output value;
in response to the initiation, receiving, from the light monitor, a measurement of light generated by the light source; and
when the measurement deviates from an expected light output value by more than a threshold amount, performing at least one of the following:
sending a warning; or
taking a corrective action.

## Patentansprüche

1. Optisches Stimulationssystem, aufweisend:
eine implantierbare Lichtquelle (120), die konfiguriert ist, Licht für eine optische Stimulation zu erzeugen;
einen implantierbaren Lichtmonitor (124);
einen implantierbaren optischen Leiter (122), der mit der Lichtquelle und dem Lichtmonitor gekoppelt oder koppelbar ist; und
ein implantierbares Steuermodul (446), das mit der Lichtquelle und dem Lichtmonitor gekoppelt oder koppelbar ist, wobei das Steuermodul aufweist:
einen Speicher (105) und
einen Prozessor (104), der mit dem Speicher gekoppelt ist und konfiguriert ist, um
eine Verifikation oder Messung eines Lichtausgabewerts automatisch zu initiieren;
als Antwort auf die Initiation, einen Messwert des von der Lichtquelle erzeugten Lichts aus dem Lichtmonitor zu empfangen; und
wenn der Messwert um mehr als einen Schwellenbetrag von einem erwarteten Lichtausgabewert abweicht, Senden einer Warnung und/oder eine korrigierende Aktion durchzuführen.

2. Optisches Stimulationssystem nach Anspruch 1, wobei der Prozessor des Weiteren konfiguriert ist, den Lichtmonitor anzuweisen, die Messung durchzuführen.

3. Optisches Stimulationssystem nach einem der Ansprüche 1 oder 2, wobei der Prozessor des Weiteren konfiguriert ist, die Lichtquelle anzuweisen, Licht zu erzeugen, das an einem Ort, wo Licht für eine von dem Lichtmonitor durchzuführende Messung gesammelt wird, voraussichtlich auf einem erwarteten Lichtausgabepegel ist.

4. Optisches Stimulationssystem nach einem der Ansprüche 1 bis 3, wobei der Lichtmonitor konfiguriert ist, einen Lichtausgabepegel direkt aus der Lichtquelle zu messen.

5. Optisches Stimulationssystem nach einem der Ansprüche 1 bis 4, wobei der optische Leiter des Weiteren aufweist: einen ersten optischen Wellenleiter (238), der konfiguriert ist, von der Lichtquelle erzeugtes Licht zu empfangen und das Licht aus einem distalen Abschnitt des optischen Leiters für die optische Stimulation zu emittieren, und einen zweiten optischen Wellenleiter (240), der konfiguriert ist, einen Teil des aus dem distalen Abschnitt des optischen Leiters emittierten Lichts zu empfangen und den empfangenen Teil des Lichts zu dem Lichtmonitor zu führen, wobei der Lichtmonitor konfiguriert ist, einen Lichtausgabepegel des aus dem distalen Abschnitt des optischen Leiters emittierten Lichts zu messen.

6. Optisches Stimulationssystem nach einem der Ansprüche 1 bis 5, des Weiteren aufweisend eine externe Vorrichtung (108), die für eine Kommunikation mit dem Steuermodul konfiguriert ist, wobei der Prozessor konfiguriert ist, die Warnung zu der externen Vorrichtung zu senden, und die externe Vorrichtung konfiguriert ist, als Antwort auf Empfangen der Warnung einem Nutzer eine visuelle oder akustische Meldung bereitzustellen.

7. Optisches Stimulationssystem nach Anspruch 6, wobei die externe Vorrichtung eine Programmiereinheit, ein klinischer Programmierer oder eine Patienten-Fernsteuerung ist.

8. Optisches Stimulationssystem nach einem der Ansprüche 1 bis 7, wobei Senden einer Warnung aufweist: das Steuermodul eine vibrierende oder akustische Warnung ausgeben lassen.

9. Optisches Stimulationssystem nach einem der Ansprüche 1 bis 8, wobei die korrigierende Aktion aufweist: Auffordern oder Anweisen eines Nutzers, die optische Stimulation anzupassen, wenn der Messwert um mehr als einen Schwellenbetrag von einem erwarteten Lichtausgabepegel abweicht.

10. Optisches Stimulationssystem nach einem der Ansprüche 1 bis 8, wobei die korrigierende Aktion aufweist: automatisches Anpassen der optischen Stimulation, wenn der Messwert um mehr als einen Schwellenbetrag von einem erwarteten Lichtausgabepegel abweicht.

11. Optisches Stimulationssystem nach einem der Ansprüche 1 bis 8, wobei die korrigierende Aktion aufweist: Unterbrechen der optischen Stimulation.

12. Optisches Stimulationssystem nach einem der Ansprüche 1 bis 11, wobei der Prozessor des Weiteren konfiguriert ist, wenn der Messwert um mehr als den Schwellenbetrag von dem erwarteten Lichtausgabewert abweicht, einen zweiten Messwert des von der Lichtquelle erzeugten Lichts zu empfangen, um die Abweichung zu bestätigen, wenn auch der zweite Messwert um mehr als den Schwellenbetrag von dem erwarteten Lichtausgabewert abweicht.

13. Optisches Stimulationssystem nach einem der Ansprüche 1 bis 12, wobei automatisches Initiieren einer Verifikation oder Messung eines Lichtausgabewerts aufweist: periodisches automatisches Initiieren der Verifikation oder Messung des Lichtausgabewerts.

14. Nichtflüchtiges prozessorlesbares Speichermedium, das Befehle zum Überwachen einer optischen Stimulation unter Verwendung eines optischen Stimulationssystems enthält, das eine implantierbare Lichtquelle (120), einen implantierbaren Lichtmonitor (124) und einen mit der Lichtquelle gekoppelten implantierbaren optischen Leiter (122) aufweist, wobei eine Ausführung der Befehle durch eine oder mehrere Prozessorvorrichtungen Aktionen durchführt, die aufweisen:
automatisches Initiieren einer Verifikation oder Messung eines Lichtausgabewerts;
als Antwort auf die Initiation, Empfangen, vom Lichtmonitor, eines Messwerts des von der Lichtquelle erzeugten Lichts; und
wenn der Messwert um mehr als einen Schwellenbetrag von einem erwarteten Lichtausgabewert abweicht,
Senden einer Warnung; und/oder
Durchführen einer korrigierenden Aktion.

15. Verfahren zum Überwachen einer optischen Stimulation unter Verwendung eines optischen Stimulationssystems, das eine implantierbare Lichtquelle (120), einen implantierbaren Lichtmonitor (124) und einen mit der Lichtquelle gekoppelten implantierbaren optischen Leiter (122) aufweist, wobei das Verfahren aufweist:
automatisches Initiieren einer Verifikation oder Messung eines Lichtausgabewerts;
als Antwort auf die Initiation, Empfangen, vom Lichtmonitor, eines Messwerts des von der Lichtquelle erzeugten Lichts; und
wenn der Messwert um mehr als einen Schwellenbetrag von einem erwarteten Lichtausgabewert abweicht,
Senden einer Warnung; und/oder
Durchführen einer korrigierenden Aktion.

## Revendications

1. Système de stimulation optique, comprenant :
une source de lumière implantable (120) configurée pour produire une lumière pour une stimulation optique ;
un dispositif de surveillance de lumière implantable (124) ;
un câble optique implantable (122) couplé, ou apte à être couplé, à la source de lumière et au dispositif de surveillance de lumière ; et
un module de commande implantable (446) couplé, ou apte à être couplé, à la source de lumière et au dispositif de surveillance de lumière, le module de commande comprenant
une mémoire (105), et
un processeur (104) couplé à la mémoire et configuré pour
initier automatiquement une vérification ou une mesure d'une valeur de sortie de lumière ;
en réponse à l'initiation, recevoir, à partir du dispositif de surveillance de lumière, une mesure d'une lumière générée par la source de lumière ; et
lorsque la mesure s'écarte d'une valeur de sortie de lumière prévue de plus d'une quantité seuil, réaliser au moins l'un parmi ce qui suit :
envoyer un avertissement ; ou
mettre en place une action corrective.

2. Système de stimulation optique selon la revendication 1, dans lequel le processeur est en outre configuré pour ordonner au dispositif de surveillance de lumière de faire la mesure.

3. Système de stimulation optique selon l'une quelconque des revendications 1 ou 2, dans lequel le processeur est en outre configuré pour ordonner à la source de lumière de générer une lumière qui est prévue d'être au niveau de sortie de lumière prévu sur un site où une lumière est collectée pour une mesure par le dispositif de surveillance de lumière.

4. Système de stimulation optique selon l'une quelconque des revendications 1 à 3, dans lequel le dispositif de surveillance de lumière est configuré pour mesurer un niveau de sortie de lumière directement à partir de la source de lumière.

5. Système de stimulation optique selon l'une quelconque des revendications 1 à 4, dans lequel le câble optique comprend en outre un premier guide d'ondes optique (238) configuré pour recevoir une lumière générée par la source de lumière et émettre la lumière à partir d'une partie distale du câble optique pour la stimulation optique et un second guide d'ondes optique (240) configuré pour recevoir une partie de la lumière émise à partir de la partie distale du câble optique et diriger la partie reçue de la lumière vers le dispositif de surveillance de lumière, dans lequel le dispositif de surveillance de lumière est configuré pour mesurer un niveau de sortie de lumière à partir de la lumière émise à partir de la partie distale du câble optique.

6. Système de stimulation optique selon l'une quelconque des revendications 1 à 5, comprenant en outre un dispositif externe (108) configuré pour une communication avec le module de commande, dans lequel le processeur est configuré pour envoyer l'avertissement au dispositif externe et le dispositif externe est configuré pour, en réponse à la réception de l'avertissement, fournir un message visuel ou auditif à un utilisateur.

7. Système de stimulation optique selon la revendication 6, dans lequel le dispositif externe est une unité de programmation, un programmateur clinicien ou une télécommande de patient.

8. Système de stimulation optique selon l'une quelconque des revendications 1 à 7, dans lequel l'envoi d'un avertissement comprend le fait d'amener le module de commande à émettre un avertissement vibratoire ou auditif.

9. Système de stimulation optique selon l'une quelconque des revendications 1 à 8, dans lequel l'action corrective comprend le fait d'inviter un utilisateur à ajuster, ou d'ordonner à un utilisateur d'ajuster, la stimulation optique si la mesure s'écarte de plus d'une quantité seuil par rapport à un niveau de sortie de lumière prévu.

10. Système de stimulation optique selon l'une quelconque des revendications 1 à 8, dans lequel l'action corrective comprend l'ajustement, de manière automatique, de la stimulation optique si la mesure s'écarte de plus d'une quantité seuil par rapport à un niveau de sortie de lumière prévu.

11. Système de stimulation optique selon l'une quelconque des revendications 1 à 8, dans lequel l'action corrective comprend l'interruption de la stimulation optique.

12. Système de stimulation optique selon l'une quelconque des revendications 1 à 11, dans lequel le processeur est en outre configuré pour, lorsque la mesure s'écarte de la valeur de sortie de lumière prévue de plus de la quantité seuil, recevoir, à partir du dispositif de surveillance de lumière, une seconde mesure d'une lumière générée par la source de lumière pour confirmer l'écart lorsque la seconde mesure s'écarte également de la valeur de sortie de lumière prévue de plus de la quantité seuil.

13. Système de stimulation optique selon l'une quelconque des revendications 1 à 12, dans lequel l'initiation, de manière automatique, d'une vérification ou d'une mesure d'une valeur de sortie de lumière comprend l'initiation, de manière automatique et périodique, de la vérification ou de la mesure de la valeur de sortie de lumière.

14. Support de stockage non transitoire lisible par processeur qui inclut des instructions pour la surveillance d'une stimulation optique à l'aide d'un système de stimulation optique comprenant une source de lumière implantable (120), un dispositif de surveillance de lumière implantable (124), et un câble optique implantable (122) couplé à la source de lumière, dans lequel l'exécution des instructions par un ou plusieurs dispositifs processeurs réalise des actions, comprenant :
l'initiation, manière automatique, d'une vérification ou d'une mesure d'une valeur de sortie de lumière ;
en réponse à l'initiation, la réception, à partir du dispositif de surveillance de lumière, d'une mesure d'une lumière générée par la source de lumière ; et
lorsque la mesure s'écarte d'une valeur de sortie de lumière prévue de plus d'une quantité seuil, la réalisation d'au moins l'un parmi ce qui suit :
l'envoi d'un avertissement ; ou
la mise en place d'une action corrective.

15. Procédé de surveillance de stimulation optique à l'aide d'un système de stimulation optique comprenant une source de lumière implantable (120), un dispositif de surveillance de lumière implantable (124), et un câble optique implantable (122) couplé à la source de lumière, le procédé comprenant :
l'initiation, manière automatique, d'une vérification ou une mesure d'une valeur de sortie de lumière ;
en réponse à l'initiation, la réception, à partir du dispositif de surveillance de lumière, d'une mesure d'une lumière générée par la source de lumière ; et
lorsque la mesure s'écarte d'une valeur de sortie de lumière prévue de plus d'une quantité seuil, la réalisation d'au moins l'un parmi ce qui suit :
l'envoi d'un avertissement ; ou
la mise en place d'une action corrective.
